# FASCICULE DE BREVET EUROPEEN

(11) **EP 3 373 992 B1**
(45) Date de publication et mention de la délivrance du brevet: **08.04.2020**
(21) Numéro de dépôt: 16809980.2
(22) Date de dépôt: 10.11.2016
(51) Int. Cl.: A61L 27/26, A61F 2/16, C08F 220/18, C08F 220/26, C08F 220/30

(54) **COPOLYMERE ACRYLIQUE, HYDROPHOBE, RETICULE, A BASE D'ALCOOL CINNAMIQUE, POUR LENTILLES INTRAOCULAIRES**
HYDROPHOBES VERNETZTES ACRYLCOPOLYMER AUF DER BASIS VON ZIMTALKOHOL FÜR INTRAOKULARLINSEN
ACRYLIC COPOLYMER, WHICH IS HYDROPHOBIC, CROSS-LINKED AND BASED ON CINNAMIC ALCOHOL, FOR INTRAOCULAR LENSES

(30) Priorité: 10.11.2015 FR 1560725
(43) Date de publication de la demande: 19.09.2018
(73) Titulaire: Acrylian, 67100 Strasbourg (FR)
(72) Inventeur: TERRISSE, Jean, 67100 Strasbourg (FR)
(74) Mandataire: Merckling, Norbert
(86) Numéro de dépôt international: PCT/FR2016/052934
(87) Numéro de publication internationale: WO 2017/081425

(56) Documents cités:
- WO-A1-2015/128555
- US-A- 5 702 825
- US-I4- T 896 013

## Description

La présente invention concerne un nouveau matériau polymère acrylique, hydrophobe, viscoélastique, souple et déformable à température ambiante, parfaitement adapté pour la réalisation de lentilles intraoculaires.

L'invention concerne également un procédé de fabrication de ce matériau polymère, ainsi que des lentilles intraoculaires réalisées à partir d'un tel matériau polymère.

Les lentilles intraoculaires sont des implants ou prothèses ophtalmologiques qui sont mises en place chirurgicalement dans l'oeil de patients souffrant par exemple de cataracte, en remplacement de leur cristallin défaillant.

Lors de cette intervention chirurgicale, le chirurgien pratique une petite incision dans la cornée du patient à travers laquelle il retire le cristallin naturel malade du patient. Puis, il met en place à travers cette incision la lentille intraoculaire dans le sac cristallinien à la place du cristallin retiré.

Afin que l'intervention soit la moins traumatisante possible pour le patient et pour éviter le développement d'un astigmatisme postopératoire, l'incision réalisée dans la cornée doit être la plus petite possible.

Lors de l'intervention chirurgicale de pose de la lentille intraoculaire, la lentille est roulée sur elle-même dans un injecteur dont l'extrémité de sortie est introduite à travers l'incision jusqu'au sac cristallinien.

La lentille intraoculaire, qui présente un diamètre nettement supérieur à la longueur de l'incision, doit être fortement comprimée pour pouvoir être expulsée à travers l'extrémité de sortie de l'injecteur dont le diamètre extérieur est inférieur ou égal à celui de l'incision.

Une fois libérée dans le sac cristallinien, la lentille intraoculaire doit se déployer rapidement et totalement pour pouvoir se positionner correctement et être capable de remplir sa fonction de correction optique de manière satisfaisante.

En raison de leur nature les destinant à être implantées à demeure à l'intérieur d'un oeil humain, de la fonction optique qu'elles doivent accomplir et de leur procédé de pose très contraignant, les lentilles intraoculaires sont soumises à de très nombreuses contraintes et doivent remplir simultanément de nombreux critères pour être jugées satisfaisantes.

D'un point de vue optique, les lentilles intraoculaires doivent être réalisées dans un matériau transparent d'indice optique suffisant, c'est-à-dire supérieur à 1,5, capable de focaliser sur la macula une fois la lentille en place, tout en ayant un encombrement minimal.

Ce matériau doit permettre de réaliser un usinage de grande précision pour obtenir la qualité optique nécessaire.

D'autre part, le matériau utilisé doit être compatible avec une implantation permanente des lentilles dans l'œil humain et ne doit pas être cytotoxique. Il ne doit pas, au fil du temps, diffuser de produits toxiques pour ne pas provoquer d'inflammation ou de nécroses.

En outre, pour que la lentille puisse être posée sans difficulté, le matériau doit être suffisamment souple pour pouvoir être plié et enroulé sur lui-même. Il doit résister à une élongation importante sous la pression de poussée sans se rompre, ni casser le tube d'injection, de manière à passer par un orifice d'éjection de diamètre extrêmement réduit, de l'ordre de 1,3 mm ou même moins de 1 mm.

Enfin, une fois dans l'oeil du patient, la lentille intraoculaire doit être capable de se déployer toute seule en quelques secondes, sans rester collée sur elle-même, afin de se positionner correctement dans le sac cristallinien et de retrouver ses caractéristiques optiques sans trace de plicature.

De nombreuses lentilles intraoculaires, de forme et de composition variées, ont été proposées dans l'art antérieur. Cependant, malgré la très grande diversité proposée, aucune n'a réussi jusqu'à présent à remplir l'ensemble de ces critères de façon satisfaisante.

Le but de l'invention est de fournir un nouveau matériau permettant la réalisation de lentilles intraoculaires remplissant l'ensemble de ces conditions.

Dans l'art antérieur, on a tenté de développer des matériaux plus déformables pour réaliser des lentilles intraoculaires plus faciles à introduire à travers une incision de plus en plus petite.

Des lentilles en matières plastiques dites « hydrophiles » ont ainsi été proposées. Bien que plus facilement déformables, elles posent des problèmes d'inflammation de l'œil, du fait de la diffusion de produits s'échappant de ces lentilles difficilement purifiables et toujours en équilibre avec l'eau de l'oeil dans lequel elles sont implantées.

En outre, les matériaux hydrophiles, tels que les hydrogels classiquement utilisés pour cette application, accélèrent la migration des cellules épithéliales sur la surface des lentilles et peuvent ainsi être responsables à long terme d'une opacification capsulaire particulièrement gênante pour le patient.

On s'est alors tourné vers les matières plastiques dites « hydrophobes » qui sont définies de manière conventionnelle par une reprise en eau inférieure à 5% à 35°C et qui présentent des caractéristiques propres qui ne dépendent pas de la quantité d'eau absorbée. Lors de la fabrication, elles peuvent être facilement purifiées et débarrassées des produits extractibles, insolubles ou peu solubles dans l'eau.

Il s'agit par exemple de polymères acryliques ou à base de silicone.

La souplesse de ces matériaux dépend de la température à laquelle ils se trouvent. Ils présentent une température de transition vitreuse (Tg) en dessous de laquelle ils sont durs et peuvent être usinés et au-dessus de laquelle ils deviennent souples, déformables et élastiques.

Pour la réalisation de lentilles intraoculaires, on doit choisir un matériau présentant une température de transition vitreuse suffisamment basse pour que la lentille résultante soit assez souple pour être roulée et étirée à la température d'une salle d'opération, soit environ 18 à 20°C.

L'invention se place dans le cadre de ces matières plastiques dites « hydrophobes » et vise plus spécifiquement les polymères acryliques.

Le problème bien connu de ces matériaux hydrophobes est que plus ils sont souples et déformables et plus ils deviennent collants.

De ce fait, les lentilles intraoculaires peuvent avoir du mal à se déployer correctement lorsqu'elles sont implantées dans l'oeil du patient. En particulier, leurs haptiques restent très souvent collées à la partie optique de la lentille.

Pour résoudre ce problème technique, on a proposé dans l'art antérieur de traiter la surface des lentilles, postérieurement à leur fabrication, pour les rendre plus glissantes et moins collantes. La demande de brevet WO 94/25510 propose par exemple d'exposer pour cela leur surface à un plasma.

On a également tenté de proposer des matériaux qui seraient par nature souples et non collants.

On connait ainsi par exemple les copolymères à base d'acrylates et de composés silicones décrits dans le brevet US 8969429 B2.

On connait également le brevet EP 2.285.427 qui décrit un matériau polymère acrylique hydrophobe pour la réalisation de lentilles intraoculaires, qui est réticulé et obtenu par polymérisation radicalaire.

Afin d'améliorer pour ce matériau l'aptitude à la déformation sans rupture aux températures de pose de la lentille, un agent de transfert de type thiol a été ajouté au mélange de monomères avant leur polymérisation. Lors de la polymérisation, cet agent de transfert interrompt localement la formation du réseau tridimensionnel pour créer des chaînes pendantes. Cela permet d'obtenir un maillage plus lâche capable de s'étirer davantage sans rupture pour un taux de réticulation et une élasticité élevés.

L'ajout au mélange de cet agent de transfert permet ainsi de conserver un taux de réticulation élevé, ce qui contribue à limiter le caractère collant du polymère résultant, tout en gardant un allongement à la rupture important.

Mais même si ce matériau polymère possède des qualités indéniables par rapport aux autres matériaux disponibles sur le marché, il présente encore des inconvénients qui l'empêchent de résoudre le problème technique de façon satisfaisante.

Ce matériau est particulièrement délicat à fabriquer. L'étape de polymérisation radicalaire nécessaire à sa formation est fortement exothermique et la vitesse de réaction doit être contrôlée. La polymérisation doit être menée avec précaution et sous surveillance constante, afin d'éviter un emballement de la réaction qui peut être dangereux et qui conduit à l'obtention d'un produit de qualité dégradée.

En effet, avec un agent de transfert tel qu'un thiol les radicaux produits par les composés initiateurs peroxydes sont transférés de la chaîne polymère interrompue aux monomères restants. Les radicaux s'accumulent de plus en plus dans le mélange réactionnel, au fur et à mesure de la décomposition des peroxydes qui s'accélère avec la hausse de la température provoquée par l'exothermie de la réaction, et la réaction peut s'emballer.

Avec ce type de compositions, on constate l'apparition d'un pic d'exothermie pouvant par exemple aller jusqu'à 180°C pour une consigne initiale de polymérisation à 80°C.

Afin de contrôler la vitesse de réaction, l'homme du métier est contraint de réaliser cette polymérisation à basse température (de 50 à 75°C) et au moyen de deux peroxydes différents : un premier peroxyde rapide agissant à basse température jusqu'à dépasser une zone critique de possible emballement correspondant à environ 10 à 15% de conversion, puis un second peroxyde plus lent qui agit à une température plus élevée pour terminer la réaction.

Toutes ces précautions, en particulier la faible température réactionnelle devant être maintenue en début de réaction, expliquent que cette étape de fabrication dure particulièrement longtemps, à savoir généralement entre 10 et 15 heures pour la polymérisation. Cette durée importante constitue un inconvénient majeur pour l'industrialisation de la production d'un tel matériau, car elle limite fortement la productivité générale pouvant être obtenue. Il est en effet difficile de réaliser plus d'un cycle de production par jour.

En outre, les thiols utilisés comme agents de transfert dans ce brevet antérieur sont des composés chimiques soufrés particulièrement désagréables à utiliser en raison de leur forte odeur nauséabonde.

Un but de l'invention est d'offrir une alternative à l'utilisation de ces thiols, et plus généralement à l'utilisation des agents de transfert, pour fabriquer un matériau polymère acrylique adapté à la réalisation de lentilles intraoculaires qui conserverait les avantages de ce matériau décrit dans l'art antérieur.

Un autre but de l'invention est de fournir un matériau nouveau qui pourrait être fabriqué plus simplement et beaucoup plus rapidement, sans présenter les inconvénients précédemment cités.

Il ne s'agit donc pas de trouver un simple équivalent à l'agent de transfert, mais d'obtenir un matériau différent bien plus avantageux.

L'invention offre ainsi une solution différente au problème technique en enseignant un nouveau matériau polymère acrylique, souple et déformable élastiquement à température ambiante, hydrophobe et réticulé, pour la réalisation de lentilles intraoculaires, qui est obtenu en une seule étape de polymérisation radicalaire, sans utiliser d'agent de transfert, et qui est beaucoup plus pratique à fabriquer, avec une productivité nettement supérieure.

Pour cela l'invention fournit un matériau polymère acrylique, hydrophobe, adapté à la réalisation de lentilles intraoculaires qui se caractérise en ce qu'il s'agit d'un copolymère réticulé, viscoélastique, souple et déformable à température ambiante, d'un mélange de monomères comprenant au moins 50% en masse de monomères acryliques ou méthacryliques et d'alcool cinnamique.

Le matériau polymère selon l'invention est un matériau acrylique, c'est-à-dire qu'il est formé majoritairement à partir de monomères acryliques et/ou méthacryliques.

Il s'agit d'un copolymère formé à partir d'un mélange de monomères contenant, en plus de l'alcool cinnamique, au moins 50%, préférentiellement au moins 80%, et encore plus préférentiellement au moins 90%, en masse d'un ou plusieurs monomères acryliques ou méthacryliques.

Ce mélange de monomères comprend
- entre 50 et 90% en masse de 2-phénoxy-éthyl acrylate (2PEA), ou d'un mélange de 2-phénoxy-éthyl acrylate (2PEA) et de 2-phénoxy-(2-éthoxy)4-acrylate (4PEA) ;
- entre 8 et 35% en masse d'un mélange d'acrylate hydroxylé et de méthacrylate hydroxylé, l'acrylate hydroxylé étant un monoacrylate de dihydroxy-alkyle ou un monoacrylate de dihydroxy-éthoxy-alkyle dont la chaîne alkyle du glycol comporte de 2 à 6 atomes de carbone, le 4-hydroxy-butyl-acrylate, l'hydroxy-éthyl-acrylate, le monoacrylate d'hexanediol, ou le monoacrylate de triéthylène glycol, et le méthacrylate hydroxylé étant un monométhacrylate de dihydroxy-alkyle ou un monométhacrylate de dihydroxy-éthoxy-alkyle dont la chaîne alkyle du glycol comporte de 2 à 6 atomes de carbone, l'hydroxy-éthyl-méthacrylate, le monométhacrylate de propanediol, le monométhacrylate de butanediol, le monométhacrylate d'hexanediol ou le monométhacrylate de triéthylène glycol;
- entre 1 et 3% en masse d'un mélange de diacrylate de diol éthoxylé et de diméthacrylate de diol éthoxylé, qui sont des diesters de polyéthylène glycol comportant de 2 à 5 fonctions éthoxyles; et
- entre 0,1% et 5% en masse d'alcool cinnamique.

Par alcool cinnamique on entend le composé chimique de formule ci-dessous :

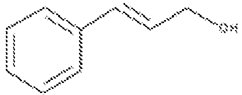

Dans la littérature, on trouve de nombreuses autres appellations pour désigner ce composé, en particulier : 3-phényl-2-propèn-1-ol, 3-phényl-2-propènol, phényl-3-propène-2-ol-1, 2-propèn-1-ol 3-phényl, alcool styrolique, alcool cinnamylique, styrone, alcool styrylique, alcool phényl-3 allylique, alcool 3-phényl-allylique, alcool γ-phényl-allyle, vinyl-phényl-carbinol, styryl-carbinol.

De façon surprenante, et alors qu'il ne comporte pas de thiol ou d'autre agent de transfert, le polymère réticulé obtenu est viscoélastique, souple et déformable élastiquement, c'est-à-dire de façon réversible, à température ambiante, sans être collant. Il peut se déformer facilement et sans rupture aux températures de pose de la lentille, et peut donc être roulé et étiré sans problème pour être implanté dans l'œil d'un patient.

Dans cette demande de brevet, on considère comme souple et déformable à température ambiante, un matériau dont la température de transition vitreuse (Tg) est inférieure à 15°C et dont le module élastique à 25°C est inférieur à 5 MPa après un temps de relaxation supérieur à 30 secondes.

On constate que de manière inattendue la présence d'alcool cinnamique parmi les monomères, permet de diminuer le module d'élasticité du copolymère réticulé obtenu, à taux de réticulant constant. L'alcool cinnamique permet ainsi d'obtenir un réseau macromoléculaire tridimensionnel qui présente un allongement à la rupture important même avec un taux de réticulation élevé, et ce alors qu'aucun agent de transfert n'est présent dans le mélange.

L'alcool cinnamique permet d'obtenir un résultat similaire à celui des composés de type thiols de l'art antérieur, mais agit d'une façon complètement différente.

En effet, contrairement aux composés de type thiol de l'art antérieur, l'alcool cinnamique est un monomère qui, pour les molécules ayant réagi, se lie au réseau macromoléculaire en formation. Il ne s'agit donc pas d'un agent de transfert qui reste libre dans le mélange réactionnel et ne fait pas partie du polymère formé.

En outre, alors les agents de transfert captent les radicaux d'une façon provisoire pour les transférer d'un macromère, réticulé ou non, vers un autre monomère, déplaçant ainsi le lieu de la polymérisation sans diminuer le nombre de radicaux libres en circulation dans le mélange réactionnel, le monomère alcool cinnamique semble fonctionner selon un mécanisme différent.

On constate que l'exothermie de la réaction reste limitée et qu'il n'y a plus de pic d'exothermie dangereux. La réaction ne risque plus de s'emballer.

Le monomère alcool cinnamique semble capter les radicaux libres macromères de manière durable et stable, conduisant ainsi à une diminution progressive du nombre de macroradicaux libres présents dès lors qu'ils sont fixés. Un tel mécanisme est compatible avec le constat que l'on ne consomme que très peu de alcool cinnamique introduit dans le mélange.

La polymérisation peut donc être réalisée sans précaution particulière, avec un unique peroxyde (peroxyde rapide en quantité suffisante) et à une température permettant une conversion totale en quelques heures (par exemple de l'ordre de 90 à 110°C), sans pic d'exothermie, ni risque d'emballement.

A une telle température, la durée de cette polymérisation peut être limitée à environ 2 à 6 heures, ce qui est largement inférieur aux durées de 10 à 15 heures nécessaires avec les agents de transfert de l'art antérieur. La productivité de fabrication s'en trouve considérablement améliorée.

En outre, cette fabrication est moins délicate et complexe à mettre en œuvre pour les opérateurs, du fait des précautions à prendre qui sont beaucoup moins critiques.

Cette nette amélioration de la productivité, couplée à la simplification de la mise œuvre du procédé de fabrication, est un avantage considérable pour l'industrialisation de la fabrication du matériau selon l'invention par rapport aux matériaux de l'art antérieur.

D'autre part, l'alcool cinnamique est beaucoup plus agréable à utiliser que les composés soufrés utilisés dans l'art antérieur, car il ne présente pas leur odeur nauséabonde. Le confort des opérateurs est ainsi amélioré.

L'alcool cinnamique n'est donc pas un simple équivalent de l'agent de transfert, car il est de nature différente, agit de façon différent et procure en outre des avantages considérables lors de la fabrication.

L'invention fournit également une lentille intraoculaire, à implanter chirurgicalement dans le sac cristallinien d'un patient en remplacement de son cristallin naturel, qui est avantageusement réalisée à partir d'un matériau polymère acrylique tel qu'exposé ci-dessus.

L'invention enseigne enfin un procédé de fabrication de ce matériau polymère acrylique qui comporte les étapes suivantes :
- on réalise un mélange de monomères contenant
   - entre 50 et 90% en masse de 2-phénoxy-éthyl acrylate (2PEA), ou d'un mélange de 2-phénoxy-éthyl acrylate (2PEA) et de 2-phénoxy-(2-éthoxy)4-acrylate (4PEA) ;
   - entre 8 et 35% en masse d'un mélange d'acrylate hydroxylé et de méthacrylate hydroxylé, l'acrylate hydroxylé étant un monoacrylate de dihydroxy-alkyle ou un monoacrylate de dihydroxy-éthoxy-alkyle dont la chaîne alkyle du glycol comporte de 2 à 6 atomes de carbone, le 4-hydroxy-butyl-acrylate, l'hydroxy-éthyl-acrylate, le monoacrylate d'hexanediol, ou le monoacrylate de triéthylène glycol, et le méthacrylate hydroxylé étant un monométhacrylate de dihydroxy-alkyle ou un monométhacrylate de dihydroxy-éthoxy-alkyle dont la chaîne alkyle du glycol comporte de 2 à 6 atomes de carbone, l'hydroxy-éthyl-méthacrylate, le monométhacrylate de propanediol, le monométhacrylate de butanediol, le monométhacrylate d'hexanediol ou le monométhacrylate de triéthylène glycol;
   - entre 1 et 3% en masse d'un mélange de diacrylate de diol éthoxylé et de diméthacrylate de diol éthoxylé, qui sont des diesters de polyéthylène glycol comportant de 2 à 5 fonctions éthoxyles; et
   - entre 0,1% et 5% en masse d'alcool cinnamique.
- on ajoute audit mélange au moins un composé initiateur ;
- on polymérise ledit mélange par voie radicalaire, en une seule étape de polymérisation, de manière à obtenir par cette polymérisation un copolymère acrylique ou méthacrylique réticulé, viscoélastique, souple et déformable à température ambiante et comportant de l'alcool cinnamique.

Selon un mode de réalisation préférentiel de l'invention, ce procédé comprend en outre une étape de purification, de préférence une lixiviation, permettant de réduire la quantité de produits extractibles dans le produit final.

D'autres caractéristiques et avantages de l'invention apparaîtront à la lecture de la description détaillée qui va suivre, en particulier le rôle, la nature préférentielle et la quantité de chacun des monomères et autres constituants du mélange permettant d'obtenir le matériau selon l'invention.

Pour faciliter la bonne compréhension du lecteur, cette description est accompagnée à titre d'exemple des dessins annexés suivants :

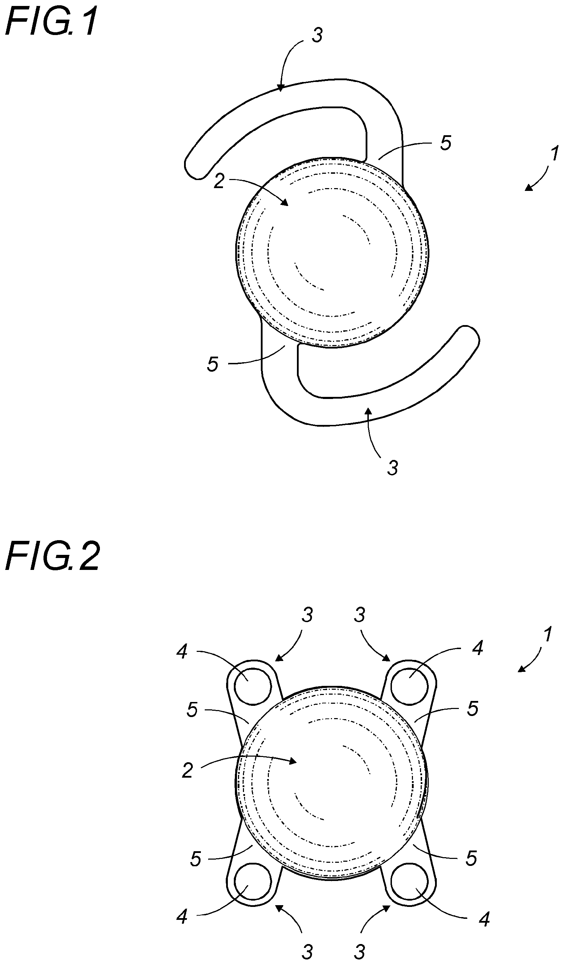

- les figures 1 et 2 sont des vues schématiques de deux exemples classiques de lentille intraoculaire pouvant être réalisée à partir du matériau selon l'invention.

Ces lentilles 1 comportent classiquement une partie optique 2 centrale, sensiblement en forme de disque et à profil le plus souvent bi-convexe. Cette partie optique 2 correctrice, doit être positionnée perpendiculairement et de manière centrée par rapport à l'axe optique de l'œil.

De cette partie optique 2 s'étendent des prolongements latéraux appelés haptiques 3, dont le rôle est de tendre les parois du sac cristallinien et d'assurer un positionnement correct de la lentille par rapport à celles-ci.

Sur la figure 1, ces haptiques 3 sont au nombre de deux. Elles sont disposées de manière diamétralement opposée et présentent une forme de bras recourbé allant chacun dans un sens opposé.

La lentille de la figure 2 comporte quatre haptiques 3 en forme d'anneau percé d'un orifice central 4. Ces haptiques 3 sont régulièrement réparties sur le pourtour de la partie optique 2.

Les haptiques 3 sont reliées à la partie optique centrale 2 par une zone de jonction 5 formant charnière qui génère un effet de ressort par rappel élastique de la matière pour déplier la lentille lors de son implantation dans l'oeil d'un patient.

Sur les exemples représentés, les haptiques 3 sont réalisées d'une pièce avec la partie optique 2 de la lentille 1. Ce type de lentille est appelé « lentille monobloc ».

Le matériau selon l'invention est particulièrement adapté à la réalisation de telles lentilles intraoculaires 1.

L'inventeur a constaté avec étonnement que le monomère d'alcool cinnamique semble jouer un rôle singulier, mais avec une réactivité avantageusement très faible, dans les compositions de monomères acryliques et/ou méthacryliques.

En procédant à la polymérisation de nombreuses compositions contenant des monomères acryliques et/ou méthacryliques et de l'alcool cinnamique en vue de l'obtention de copolymères réticulés, il a fait plusieurs observations étonnantes, à savoir que :
- le monomère d'alcool cinnamique était peu consommé par rapport à la masse introduite,
- la cinétique de la réaction était modifiée au point de supprimer quasiment le pic d'exothermie qui était très présent avec les agents de transfert,
- la nature chimique du produit obtenu n'est que très faiblement modifiée par la présence de monomères d'alcool cinnamique, et
- les propriétés mécaniques du produit obtenu sont profondément modifiées et ce malgré la faible quantité consommée de monomères d'alcool cinnamique.

A partir de ces constats, l'inventeur a proposé l'interprétation générale, cohérente avec chacune de ces observations, que le monomère d'alcool cinnamique semblait jouer un rôle de terminaison de chaîne, mais avec une réactivité avantageusement très faible.

En effet, l'absence de pic d'exothermie peut être interprétée comme liée à une disparition de radicaux (très différente de l'accumulation de radicaux provoquée par les agents de transfert de l'art antérieur), et l'incidence sur les propriétés mécaniques à la production de chaînes pendantes par la réaction de terminaison. La faible consommation du monomère d'alcool cinnamique et le fait qu'il se limite à une position de bout de chaîne expliquerait que les propriétés chimiques du polymère obtenu ne soient que très faiblement modifiées.

De façon surprenante, le monomère d'alcool cinnamique présente une réactivité particulière, différente de celle à laquelle l'homme du métier pourrait s'attendre par généralisation du comportement des autres membres de la famille des composés allyliques ou de la famille des styréniques.

L'inventeur a en effet testé de nombreux composés appartenant à ces familles allyliques et styréniques, notamment l'a-méthyl-styrène, le vinyl toluène, l'alcool et les esters allyliques, sans obtenir de résultats satisfaisants pour la fabrication d'un matériau polymère pour lentilles intraoculaires.

Il a constaté que, pendant l'étape de polymérisation radicalaire, ces composés allyliques ou styréniques, une fois accrochés au réseau macromoléculaire, continuent à polymériser, soit par copolymérisation avec les autres monomères ou, pour certains, en fin de réaction par homopolymérisation avec des molécules identiques, et s'insèrent ainsi à l'intérieur du réseau. La structure et la nature chimique du réseau obtenu s'en trouvent modifiées d'une façon suffisante pour changer les propriétés chimiques et physiques, notamment optiques, du matériau polymère résultant, le rendant inadapté à la réalisation de lentilles intraoculaires.

L'alcool cinnamique présente une structure particulière, comprenant un noyau aromatique conjugué avec une double liaison allylique, qui lui donne un comportement inattendu et différent de celui des produits de la même famille.

Contrairement aux autres composés allyliques, le radical créé au niveau de la double liaison de l'alcool cinnamique semble très stable et arrête la copolymérisation des espèces présentes sur la chaîne ayant capté un monomère d'alcool cinnamique. Les monomères d'alcool cinnamique sont donc peu présents au sein du réseau macromoléculaire résultant et leur influence sur les propriétés chimiques du réseau global reste limitée. Ils sont donc sans conséquence notable sur les propriétés chimiques du réseau macromoléculaire et pour la réalisation de lentilles intraoculaires.

Par contre, en interrompant localement la formation du maillage tridimensionnel, les monomères d'alcool cinnamique se comportent comme des agents de terminaison et forment à leur niveau une maille coupée avec une chaîne pendante courte reliée au réseau mais dont l'autre extrémité d'alcool cinnamique reste libre. Par la création de ces chaînes pendantes localisées, ils permettent ainsi d'obtenir un maillage plus lâche, c'est-à-dire un réseau de faible module élastique, capable de s'étirer davantage sans rupture.

Des agents de terminaison classiques, tels que des capteurs de radicaux de type stabilisants quinoniques ou quinoléines, ont également été testés. Cependant, les composés testés n'ont pas, non plus, permis d'obtenir des résultats satisfaisants pour la fabrication d'un matériau polymère pour lentilles intraoculaires.

En effet, ces composés captent très rapidement la plupart des radicaux présents dans le milieu réactionnel. Même ajoutés en très faible quantité, ils provoquent un arrêt rapide de la polymérisation qui n'aboutit qu'à la formation de quelques macromères de petite taille et non à celle du réseau macromoléculaire réticulé souhaité.

Pour pouvoir utiliser efficacement de tels composés, une quantité extrêmement faible, dosée avec beaucoup de précision, devrait être ajoutée. Ceci est bien évidemment incompatible avec la fabrication industrielle recherchée.

Avantageusement, l'alcool cinnamique n'a qu'un faible rendement en tant que comonomère. En effet, de 50 à 80% de l'alcool cinnamique introduit dans le mélange réactionnel n'est pas capté par les macromères et reste présent à l'état libre à la fin de la polymérisation.

Il peut donc être ajouté en quantité facilement mesurable et manipulable, compatible avec un procédé industrialisable, sans provoquer un arrêt complet de la polymérisation, ni conduire à la création d'un nombre de chaînes pendantes trop important.

Une fois la polymérisation terminée, les monomères d'alcool cinnamique n'ayant pas réagi et tous les autres composés monomères résiduels ou autres, non fixés au réseau, sont extraits du polymère obtenu par une simple étape de purification, par exemple une lixiviation.

Pour obtenir un résultat satisfaisant, on utilise une quantité d'alcool cinnamique comprise entre 0,1% et 5%, plus préférentiellement entre 0,2% et 2%, et de manière préférée sensiblement égale à 0,5%, en masse du mélange de monomères.

D'autres composés de la même famille que l'alcool cinnamique ont également été étudiés mais n'ont pas été retenus. L'aldéhyde cinnamique est ainsi trop toxique pour pouvoir être utilisé dans des lentilles intraoculaires. Des esters cinnamates, obtenus en estérifiant l'acide cinnamique avec divers alcools contenant de 1 à 12 carbones, ont été testés mais ne se sont pas révélés efficaces lors de la polymérisation. Ils n'ont pas permis de réduire le pic d'exothermie et n'ont pas conféré au matériau polymère résultant les propriétés attendues. De même, des esters de l'alcool cinnamique avec des acides tels que l'acide acétique, l'acide benzoïque ou leurs homologues, n'ont pas donnés d'effet notable sur la réaction comparable à celui obtenu avec l'alcool cinnamique.

En raison de son comportement inattendu, il apparait que le choix avantageux du monomère d'alcool cinnamique pour la réalisation d'un matériau polymère selon l'invention n'est pas un choix arbitraire, habituel ou évident pour l'homme du métier. Ce composé, très peu utilisé dans ce domaine technique, fait preuve d'un comportement surprenant et confère des avantages déterminants pour la fabrication du polymère.

Des exemples des autres monomères utilisés pour obtenir un matériau selon l'invention vont maintenant être détaillés.

On utilise le 2-phénoxy-éthylacrylate, communément appelé 2PEA, seul ou en mélange avec le 2-phénoxy-(2-éthoxy)₄-acrylate, usuellement appelé 4PEA.

Le 4PEA permet avantageusement de faire baisser la température de transition vitreuse du polymère résultant, tout en lui conférant une certaine hydrophilie pour faire baisser le caractère autocollant des surfaces sur elles-mêmes. Il contribue également à réduire le « glistening », c'est-à-dire la sensibilité au blanchiment du matériau polymère obtenu.

Le mélange de monomères comprend entre 50 et 90% en masse de 2PEA ou de mélange de 2PEA et de 4PEA. De préférence, il en contient entre 70 et 85% en masse.

Dans le cas d'un mélange de 2PEA et de 4PEA, on aura de préférence 65 à 75% de 2PEA et 6 à 20% de 4PEA.

Un autre exemple préférentiel de réalisation ne contient que du 2PEA.

Le mélange de monomères contient également un mélange d'acrylate hydroxylé et de méthacrylate hydroxylé.

Ces monomères hydroxylés augmentent la tension de surface et l'affinité de la surface avec l'eau du polymère résultant, ce qui diminue le caractère collant du matériau, notamment en présence d'eau. Ils contribuent également à diminuer le phénomène de « glistening ».

Ces effets sont encore augmentés par la présence du monomère d'alcool cinnamique terminant les chaînes, qui augmente la quantité globale de fonctions alcool présentes en bout de chaînes.

L'acrylate hydroxylé utilisé est un mono-acrylate de dihydroxy-alkyle ou un mono-acrylate de dihydroxy-éthoxy-alkyle dont la chaîne alkyle du glycol comporte de 2 à 6 atomes de carbone. On peut citer à titre d'exemple le 4-hydroxy-butyl-acrylate également appelé monoacrylate de butanediol ou 4HBA, l'hydroxy-éthyl-acrylate ou HEA, le monoacrylate d'hexanediol, ou encore le monoacrylate de triéthylène glycol.

Le méthacrylate hydroxylé utilisé est un monométhacrylate de dihydroxy-alkyle ou un monométhacrylate de dihydroxy-éthoxy-alkyle dont la chaîne alkyle du glycol comporte de 2 à 6 atomes de carbone. Il s'agit par exemple de l'hydroxy-éthyl-méthacrylate ou HEMA, du monométhacrylate de propanediol, du monométhacrylate de butanediol, du monométhacrylate d'hexanediol ou du monométhacrylate de triéthylène glycol.

La proportion de ces monomères hydroxylés dans le mélange avant polymérisation doit être suffisante pour que le matériau résultant présente une tension de surface convenable et ne blanchisse pas au contact prolongé de l'eau à 35°C. Elle doit cependant être limitée pour que le matériau résultant reste globalement hydrophobe et n'absorbe pas plus de 5 % d'eau à 35°C comme il est convenu pour les produits dits hydrophobes.

Avantageusement, l'acrylate hydroxylé et le méthacrylate hydroxylé représentent ensemble entre 8 et 35% en masse du mélange de monomères, et plus préférentiellement entre 15 et 30% du mélange.

La proportion relative de ces deux monomères hydroxylés l'un par rapport à l'autre peut varier selon les cas de 20 à 80% pour l'un et inversement pour l'autre en fonction de la température de transition vitreuse souhaitée.

Un exemple préférentiel de polymère selon l'invention peut ainsi comprendre par exemple de 15 à 20 % d'alcool acrylique de type HEA ou 4HBA et 4 à 8 % d'alcool méthacrylique de type HEMA.

Le mélange contient également des composés réticulants permettant d'obtenir après polymérisation un réseau macromoléculaire tridimensionnel et non des polymères linéaires. Il s'agit de monomères difonctionnels de type diacrylate de diol ethoxylé et diméthacrylate de diol éthoxylé.

Ces composés réticulants comportent préférentiellement des fonctions éthoxy de façon à ne pas augmenter la température de transition vitreuse du matériau final et simultanément à maintenir un niveau d'hydrophilie homogène avec le reste de la composition.

Le diacrylate de diol éthoxylé et le diméthacrylate de diol éthoxylé utilisés sont des diesters de polyéthylène glycol comportant de 2 à 5 fonctions éthoxyles, préférentiellement le diacrylate de tétraéthylène glycol et le diméthacrylate de tétraéthylène glycol.

Avantageusement, l'ensemble diacrylate de diol éthoxylé et diméthacrylate de diol éthoxylé représente préférentiellement entre 1 et 3% en masse du mélange de monomères, la proportion relative entre le diacrylate de diol éthoxylé et le diméthacrylate de diol éthoxylé variant de préférence de 25 à 75% de l'un par rapport à l'autre et inversement.

Il est également possible de rajouter parmi les monomères un ou plusieurs colorant(s) polymérisable(s) ou non polymérisable(s), ou un ou plusieurs agent(s) anti UV dont la fonction au sein du matériau final est d'absorber les rayonnements ultra-violets. Il peut s'agir par exemple du 2-[3-(2H-benzotriazol 2 yl)-4-hydroxyphenyl]ethyl-méthacrylate que l'on utilise préférentiellement en proportion comprise entre 0.1% et 1% en masse, et par exemple avec une teneur de 0,5%.

On obtient alors un copolymère réticulé d'au moins les monomères précédemment cités et d'un monomère absorbeur d'UV.

Tout autre monomère ou tout autre constituant polymérisable ou non polymérisable imaginable par l'homme du métier, de fonction quelconque, pourra être rajouté dans le mélange sans sortir de la présente invention, tant que sa présence ne modifie pas les propriétés générales du matériau polymère résultant d'une façon qui le rende inadapté à la réalisation de lentilles intraoculaires.

On peut citer à titre d'exemple l'ajout d'un colorant jaune, par exemple un dérivé acrylique ou méthacrylique d'un colorant polymérisable.

En plus de ces monomères, le mélange initial peut contenir un certain nombre de composés supplémentaires de nature différente, par exemple nécessaires au bon déroulement de la réaction.

Il comprend ainsi un ou plusieurs, mais préférentiellement un seul, composé initiateur qui sert à amorcer la réaction de polymérisation en créant des sites actifs sur les monomères.

Ce composé initiateur est préférentiellement un peroxyde, choisi en fonction de son temps de demi-vie devant permettre une polymérisation rapide et contrôlable dans les conditions retenues pour la polymérisation du matériau selon l'invention, et en fonction de la pureté du produit commercialement disponible.

Ce peroxyde peut être choisi parmi les acyl-peroxydes, les alkyl-peroxydes et les peroxyesters et les peroxycarbonates. Parmi ces produits, on peut citer à titre d'exemple non exclusif le tert-amyl-peroxy-2-ethyl-hexyl-carbonate également appelé Taec, le tert-butyl-peroxy-2-ethyl-hexanoate, le 1,1-ditertio-butyl-peroxy-3,3,5-trimethylcyclohexane, le 1,1-ditertio-butyl-peroxycyclohexane, le tertio-butyl-peroxy-3,3,5-trimethylhexanoate, le tertiobutyl-peroxy-isopropylcarbonate, le dicumyl-peroxide, le tert-butylcumyl-peroxide, le di-tert-amyl-peroxide, le tert-butyl-3-isopropenyl-cumyl-peroxide, le dioctananoyl-peroxide, le didecanoyl-peroxide, le tert-butyl-peroxy-2-ethylhexanoate, le tert-amyl-peroxy-2-ethylhexanoate, le tert-butyl-peroxy-3,5,5-trimethylhexanoate, 2,5-dimethyl-2,5-di(benzoylperoxy)hexane, le tert-amyl-peroxyacetate, le tert-butyl-peroxypivalate, le tert-amyl-peroxypivalate, le tert-butyl-peroxybenzoate, le tert-amyl-peroxybenzoate; le tertio-butyl-peroxy-2-ethylhexyl carbonate, le tertio-amyl peroxy-2-ethylhexyl carbonate, ou le tertio-butyl-peroxy-isobutyrate.

Ce ou ces composés sont ajoutés au mélange en très petite quantité, le mélange comprenant par exemple entre 0,3 et 2% en masse de composé initiateur.

La température de polymérisation est déterminée par le temps de demi-vie du peroxyde retenu d'une part et la durée de polymérisation visée d'autre part, de manière à cumuler typiquement 2 à 6 temps de demi-vie.

En résumant les considérations détaillées ci-dessus, on peut imaginer un mélange particulier de monomères conduisant par polymérisation radicalaire à un mode de réalisation préférentiel du matériau selon l'invention, sans toutefois limiter l'invention à celui-ci.

Ce mélange comprend préférentiellement au moins les monomères suivants : le 2-phénoxy-éthylacrylate, le 2-phénoxy(-2-éthoxy)₄-acrylate, le 4-hydroxy-butyl-acrylate et/ou l'hydroxy-éthyl-acrylate, l'hydroxy-éthyl-méthacrylate, le diacrylate de tétraéthylène glycol, le diméthacrylate de tétraéthylène glycol, et l'alcool cinnamique.

Afin de rendre ce descriptif plus complet, on va maintenant décrire un exemple de procédé d'obtention d'un matériau polymère acrylique selon l'invention à partir du mélange initial préférentiel détaillé ci-dessus.

Pour réaliser la polymérisation recherchée, on commence par mélanger entre eux tous les monomères nécessaires à la réaction, avec parmi ceux-ci l'alcool cinnamique. Avantageusement, ces monomères sont solubles les uns dans les autres et une simple agitation suffit à réaliser un mélange homogène de ceux-ci.

On ajoute ensuite à ce mélange le composé initiateur nécessaire pour déclencher la réaction de polymérisation.

On réalise ensuite la polymérisation par voie radicalaire, en une seule étape.

Il s'agit d'une polymérisation en masse, sans solvant et avec tous les réactifs présents dès le départ, avec un dosage approprié pour obtenir le polymère final avec toutes les propriétés souhaitées, en une seule opération.

Il n'est donc pas nécessaire de faire, comme dans certains exemples de l'art antérieur, un pré-polymère visqueux, moulé dans un second temps.

Pour cela, de petites quantités du mélange de monomères sont avantageusement placées dans des moules et chauffées par exemple à une température comprise entre 90 et 110°C pendant 3 à 6 heures.

Une fois la réaction terminée et après refroidissement, on procède au démoulage du polymère.

De telles durées de polymérisation permettent de réaliser plusieurs cycles quotidiens de moulage avec le même moule.

Les moules sont préférentiellement choisis de façon à obtenir après démoulage des blocs de polymère de forme générale cylindrique de faible hauteur, de type « jeton » ou « palet ». Une telle forme est parfaitement adaptée à un usinage ultérieur de ces blocs de polymère en vue d'obtenir les lentilles intraoculaires.

Bien entendu, un moulage direct des lentilles intraoculaires est également possible avec un moule adapté.

Les blocs de polymère sont ensuite purifiés, afin de les débarrasser des monomères n'ayant pas réagi, en particulier de l'alcool cinnamique, et des impuretés et produits résiduels provenant notamment de la synthèse de chacun des monomères utilisés. Cette purification peut préférentiellement se faire par lixiviation.

Après cette étape de purification, on obtient un matériau qui présente les propriétés physiques indiquées dans la présente demande.

Les blocs de matériau polymère sont alors prêts à être usinés, à une température inférieure à la température de transition vitreuse du polymère, pour réaliser les lentilles intraoculaires selon l'invention.

Afin de parfaitement décrire l'invention, plusieurs exemples de matériau polymère acrylique selon l'invention sont détaillés ci-dessous.

Les matériaux polymères acryliques ont été obtenus par polymérisation radicalaire à partir des mélanges de monomères suivants, dont les quantités sont exprimées en pourcentages massiques du mélange de monomères avant polymérisation.

Dans tous les exemples ci-dessous, le mélange de monomères a été chauffé progressivement pendant une durée de 1H30 pour passer de la température ambiante à 90°C. Il a ensuite été maintenu à 90°C pendant 1 heure pour passer de l'état liquide à l'état gel, puis chauffé à 110°C pendant 3 heures.

Aucun pic d'exothermie n'a été observé pendant toute la durée de la polymérisation, en particulier lors du passage de l'état liquide à l'état gel.

**Exemple 1 :**

| MONOMÈRES : | % massique |
|---|---|
| - 2 phénoxy-éthylacrylate | 77,2 % |
| - 2-phénoxy-(2-éthoxy)₄ acrylate | 6 % |
| - 4-hydroxy-butyl-acrylate | 9,7 % |
| - hydroxy-éthyl-méthacrylate | 4 % |
| - diacrylate de tétraéthylène glycol | 0,6 % |
| - diméthacrylate de tétraéthylène glycol | 2,0 % |
| - alcool cinnamique | 0,5 % |

La réaction de polymérisation est déclenchée par l'ajout de 0,5% de TAEC. On obtient, après polymérisation, un matériau polymère acrylique présentant un indice optique égal à 1,5381, une température de transition vitreuse égale à 7,5°C et un module élastique de 0,658 Mpa à 25°C après une minute de relaxation.

**Exemple 2 :**

| MONOMÈRES : | % massique |
|---|---|
| - 2 phénoxy-éthylacrylate | 68,7 % |
| - 2-phénoxy-(2-éthoxy)₄ acrylate | 6 % |
| - hydroxy-éthyl-acrylate | 15,3 % |
| - hydroxy-éthyl-méthacrylate | 7,7 % |
| - diacrylate de tétraéthylène glycol | 0,45 % |
| - diméthacrylate de tétraéthylène glycol | 1,35 % |
| - alcool cinnamique | 0,5 % |

La réaction de polymérisation est déclenchée par l'ajout de 0,5% de TAEC. On obtient, après polymérisation, un matériau polymère acrylique présentant un indice optique égal à 1,540, une température de transition vitreuse égale à 12,4°C et un module élastique de 0,51 Mpa à 25°C après une minute de relaxation.

**Exemple 3 :**

| MONOMÈRES : | % massique |
|---|---|
| - 2 phénoxy-éthylacrylate | 61,4 % |
| - 2-phénoxy-(2-éthoxy)₄ acrylate | 13 % |
| - 4-hydroxy-butyl-acrylate | 16,5 % |
| - hydroxy-éthyl-méthacrylate | 6,8 % |
| - diacrylate de tétraéthylène glycol | 0,45 % |
| - diméthacrylate de tétraéthylène glycol | 1,35 % |
| - alcool cinnamique | 0,5 % |

La réaction de polymérisation est déclenchée par l'ajout de 0,5% de TAEC. On obtient après polymérisation un matériau polymère acrylique présentant un indice optique égal à 1,5390, une température de transition vitreuse égale à 6,3°C et un module élastique de 0,495 Mpa à 25°C après une minute de relaxation.

**Exemple 4 :**

| MONOMÈRES : | % massique |
|---|---|
| - 2 phénoxy-éthylacrylate | 74,4 % |
| - 4-hydroxy-butyl-acrylate | 16,5 % |
| - hydroxy-éthyl-méthacrylate | 6,8 % |
| - diacrylate de tétraéthylène glycol | 0,45 % |
| - diméthacrylate de tétraéthylène glycol | 1,35 % |
| - alcool cinnamique | 0,5 % |

La réaction de polymérisation est déclenchée par l'ajout de 0,5% de TAEC. On obtient, après polymérisation un matériau polymère acrylique présentant un indice optique égal à 1,5420, une température de transition vitreuse égale à 11,2°C et un module élastique de 0,550 Mpa à 25°C après une minute de relaxation.

**Exemple 5 :**

| MONOMÈRES : | % massique |
|---|---|
| - 2 phénoxy-éthylacrylate | 72,4 % |
| - 4-hydroxy-butyl-acrylate | 17,9 % |
| - hydroxy-éthyl-méthacrylate | 7,4 % |
| - diacrylate de tétraéthylène glycol | 0,45 % |
| - diméthacrylate de tétraéthylène glycol | 1,35 % |
| - alcool cinnamique | 0,5 % |

La réaction de polymérisation est déclenchée par l'ajout de 0,5% de TAEC. On obtient, après polymérisation, un matériau polymère acrylique présentant un indice optique égal à 1,5420, une température de transition vitreuse égale à 10°C, et un module élastique de 0,510 Mpa à 25°C après une minute de relaxation.

A la lecture de tous ces exemples, on constate que pour obtenir le matériau polymère selon l'invention la durée de la polymérisation est bien plus courte que dans l'art antérieur et qu'elle se fait sans pic d'exothermie et donc sans risque d'emballement.

Afin de démontrer les propriétés surprenantes et particulièrement avantageuses conférées par l'alcool cinnamique au matériau selon l'invention concernant son aptitude à la déformation sans rupture aux températures d'utilisation, on a regroupé dans le tableau ci-dessous la composition et le module d'élasticité E, exprimé en Mpa et mesuré après 60 secondes à une température de 25°C, de différents matériaux polymères acryliques, destinés à la réalisation de lentilles intraoculaires.

| **N° de la formule testée** | **Composition en pourcentage massique** | | | | | | | | **Module d'élasticité E en Mpa, après 60 s à 25°C** |
|---|---|---|---|---|---|---|---|---|---|
| | 2PEA | 4PEA | 4HBA | HEMA | TDIA | TDIMA | AC | Thiol | |
| 1 | 77.6 | 6,0 | 9,7 | 4,0 | 0,6 | 2,0 | 0,1 | | **0,814** |
| 2 | 77,2 | 6,0 | 9,7 | 4,0 | 0,6 | 2,0 | 0,5 | | **0,658** |
| 3 | 76,7 | 6,0 | 9,7 | 4,0 | 0,6 | 2,0 | 1,0 | | **0,561** |
| 4 | 74,7 | 6,0 | 9,7 | 4,0 | 0,6 | 2,0 | 3,0 | | **0,460** |
| 5 | 72,7 | 6,0 | 9,7 | 4,0 | 0,6 | 2,0 | 5,0 | | **0,348** |
| 6 | 77,7 | 6,0 | 9,7 | 4,0 | 0,6 | 2,0 | | 0,1 | **0,819** |
| 7 | 77,7 | 6,0 | 9,7 | 4,0 | 0,6 | 2,0 | | | **≈ 0,90*** |

Les formules 1 à 5 correspondent à des matériaux selon l'invention contenant de l'alcool cinnamique (AC) dans leur composition.

La formule 6 correspond à une formule ne contenant pas d'alcool cinnamique, mais dans laquelle selon l'art antérieur un agent de transfert de type thiol a été ajouté dans le mélange de monomères.

La formule 7 correspond à un matériau de référence ne contenant ni alcool cinnamique, ni thiol.

Les abréviations utilisées dans le tableau ont la signification suivante :
- 2PEA :: 2 phénoxy-éthylacrylate
- 4PEA :: 2-phénoxy-(2-éthoxy)4 acrylate
- 4HBA :: 4-hydroxy-butyl-acrylate
- HEMA :: hydroxy-éthyl-méthacrylate
- TDIA :: diacrylate de tétraéthylène glycol
- TDIMA :: diméthacrylate de tétraéthylène glycol
- AC :: alcool cinnamique
- * :: Matériau écarté de l'étude, car il présente de nombreuses fissures et est inadapté à la réalisation de lentilles intraoculaires. Son module d'élasticité n'a pas pu être mesuré avec précision.

Afin de pouvoir évaluer l'influence de l'alcool cinnamique sur l'élasticité du matériau résultant, les différentes formules testées ont une composition très similaire. La proportion relative des monomères est identique, à l'exception de l'alcool cinnamique et de la 2PEA qui varie en conséquence pour ajuster le total des monomères à 100%. La formule n° 6 contient un thiol en plus des monomères.

En observant le tableau, on constate que plus on augmente la quantité d'alcool cinnamique et plus le module d'élasticité du matériau obtenu diminue, alors que le taux de réticulant est par ailleurs maintenu constant (même quantités de TDIA et TDIMA utilisées).

L'ajout d'alcool cinnamique en milieu acrylique permet donc bien d'améliorer, d'adapter ou d'ajuster l'élasticité du matériau polymère obtenu, sans être obligé de diminuer le taux de réticulant.

Pour la formule 1, cet effet est similaire à celui obtenu avec l'agent de transfert de type thiol dans la formule 6. Il est même plus important dans toutes les autres formules 2 à 5 testées.

On constate également que l'ajout d'alcool cinnamique améliore considérablement la situation par rapport à la formule n°7. En effet, avec la formule 7 de référence ne contenant ni alcool cinnamique ni thiol, on a obtenu un produit extrêmement cassant, qui n'a pas supporté les contraintes provoquées par le retrait volumique lié à la polymérisation dans les conditions choisies et dans lequel de nombreuses fissures se sont formées, le rendant inapproprié à l'application visée.

## Revendications

1. Matériau polymère acrylique, hydrophobe c'est-à-dire n'absorbant pas plus de 5% d'eau à 35°C, adapté pour la réalisation de lentilles intraoculaires souples, **caractérisé en ce qu'**il s'agit d'un copolymère réticulé, viscoélastique, souple et déformable à température ambiante, préparé à partir d'un mélange de monomères comprenant :
- entre 50 et 90% en masse de 2-phénoxy-éthyl acrylate (2PEA), ou d'un mélange de 2-phénoxy-éthyl acrylate (2PEA) et de 2-phénoxy-(2-éthoxy)₄-acrylate (4PEA) ;
- entre 8 et 35% en masse d'un mélange d'acrylate hydroxylé et de méthacrylate hydroxylé, l'acrylate hydroxylé étant un monoacrylate de dihydroxy-alkyle ou un monoacrylate de dihydroxy-éthoxy-alkyle dont la chaîne alkyle du glycol comporte de 2 à 6 atomes de carbone, le 4-hydroxy-butyl-acrylate, l'hydroxy-éthyl-acrylate, le monoacrylate d'hexanediol, ou le monoacrylate de triéthylène glycol, et le méthacrylate hydroxylé étant un monométhacrylate de dihydroxy-alkyle ou un monométhacrylate de dihydroxy-éthoxy-alkyle dont la chaîne alkyle du glycol comporte de 2 à 6 atomes de carbone, l'hydroxy-éthyl-méthacrylate, le monométhacrylate de propanediol, le monométhacrylate de butanediol, le monométhacrylate d'hexanediol ou le monométhacrylate de triéthylène glycol;
- entre 1 et 3% en masse d'un mélange de diacrylate de diol éthoxylé et de diméthacrylate de diol éthoxylé, qui sont des diesters de polyéthylène glycol comportant de 2 à 5 fonctions éthoxyles; et
- entre 0,1% et 5% en masse d'alcool cinnamique.

2. Matériau polymère acrylique selon la revendication 1 **caractérisé en ce que** le mélange de monomères comprend au moins 80%, préférentiellement au moins 90%, en masse de monomères acryliques ou méthacryliques.

3. Matériau polymère acrylique selon la revendication 1 ou 2 **caractérisé en ce que** le 2-phénoxy-éthyl acrylate (2PEA), ou le mélange de 2-phénoxy-éthyl acrylate (2PEA) et de 2-phénoxy-(2-éthoxy)₄-acrylate (4PEA) représente entre 70 et 85% en masse du mélange de monomères.

4. Matériau polymère acrylique selon la revendication 1 **caractérisé en ce que** le mélange de monomères comprend un mélange de 2-phénoxy-éthyl acrylate (2PEA) et de 2-phénoxy-(2-éthoxy)₄-acrylate (4PEA) avec de 65 à 75% de 2PEA et 6 à 20% de 4PEA.

5. Matériau polymère acrylique selon l'une quelconque des revendications précédentes **caractérisé en ce que** l'acrylate hydroxylé et le méthacrylate hydroxylé représentent ensemble entre 15 et 30% en masse du mélange de monomères.

6. Matériau polymère acrylique selon l'une quelconque des revendications précédentes **caractérisé en ce que** le diacrylate de diol éthoxylé et le diméthacrylate de diol éthoxylé sont le diacrylate de tétraéthylène glycol et le diméthacrylate de tétraéthylène glycol.

7. Matériau polymère acrylique selon l'une quelconque des revendications précédentes **caractérisé en ce que** l'alcool cinnamique représente entre 0,2% et 2%, et plus préférentiellement environ 0,5%, en masse du mélange de monomères.

8. Matériau polymère acrylique selon l'une quelconque des revendications précédentes **caractérisé en ce que** le mélange de monomères comprend en outre un monomère absorbeur d'UV.

9. Procédé de fabrication d'un matériau polymère acrylique selon l'une quelconque des revendications 1 à 8 **caractérisé en ce qu'**il comporte les étapes suivantes :
• on réalise un mélange de monomères contenant :
- entre 50 et 90% en masse de 2-phénoxy-éthyl acrylate (2PEA), ou d'un mélange de 2-phénoxy-éthyl acrylate (2PEA) et de 2-phénoxy-(2-éthoxy)₄-acrylate (4PEA) ;
- entre 8 et 35% en masse d'un mélange d'acrylate hydroxylé et de méthacrylate hydroxylé, l'acrylate hydroxylé étant un monoacrylate de dihydroxy-alkyle ou un monoacrylate de dihydroxy-éthoxy-alkyle dont la chaîne alkyle du glycol comporte de 2 à 6 atomes de carbone, le 4-hydroxy-butyl-acrylate, l'hydroxy-éthyl-acrylate, le monoacrylate d'hexanediol, ou le monoacrylate de triéthylène glycol, et le méthacrylate hydroxylé étant un monométhacrylate de dihydroxy-alkyle ou un monométhacrylate de dihydroxy-éthoxy-alkyle dont la chaîne alkyle du glycol comporte de 2 à 6 atomes de carbone, l'hydroxy-éthyl-méthacrylate, le monométhacrylate de propanediol, le monométhacrylate de butanediol, le monométhacrylate d'hexanediol ou le monométhacrylate de triéthylène glycol;
- entre 1 et 3% en masse d'un mélange de diacrylate de diol éthoxylé et de diméthacrylate de diol éthoxylé, qui sont des diesters de polyéthylène glycol comportant de 2 à 5 fonctions éthoxyles; et
- entre 0,1% et 5% en masse d'alcool cinnamique
• on ajoute audit mélange au moins un composé initiateur ;
• on polymérise ledit mélange par voie radicalaire, en une seule étape de polymérisation, de manière à obtenir par cette polymérisation un copolymère acrylique ou méthacrylique réticulé, viscoélastique, souple et déformable à température ambiante, et comportant de l'alcool cinnamique.

10. Procédé de fabrication selon la revendication 9 **caractérisé en ce qu'**il comprend en outre une étape de purification.

11. Lentille intraoculaire souple à implanter chirurgicalement dans le sac cristallinien d'un patient en remplacement de son cristallin naturel, **caractérisée en ce qu'**elle est réalisée à partir d'un matériau polymère acrylique selon l'une quelconque des revendications 1 à 8.

## Patentansprüche

1. Hydrophobes Acrylpolymer-Material, also mit einer Wasserabsorption von weniger als 5 % bei 35°C, das für die Herstellung weicher Intraokularlinsen geeignet ist, **dadurch gekennzeichnet, dass** es sich um ein vernetztes, viskoelastisches, flexibles und bei Raumtemperatur verformbares Material handelt, das aus einer Monomermischung hergestellt wird, die enthält:
- zwischen 50 und 90 Masse-% 2-phenoxyethylacrylat (2PEA) oder eine Mischung aus 2-phenoxyethylacrylat (2PEA) und 2-phenoxy-(2-ethoxy)₄-Acrylat (4PEA);
- zwischen 8 und 35 Masse-% einer Mischung aus hydroxylgruppenhaltigem Acrylat und hydroxylgruppenhaltigem Methacrylat, wobei es sich bei dem hydroxylgruppenhaltigen Acrylat um ein Dihydroxyalkyl-Monoacrylat oder ein Dihydroxy-Ethoxyalkyl-Monoacrylat handelt, dessen Glykol-Alkylgruppe 2 bis 6 Kohlenstoffatome enthält, wobei es sich bei dem 4-hydroxy-butyl-acrylat, dem Hydroxy-ethyl-acrylat, dem Hexandiol-Monoacrylat oder dem Triethylen-Glycolmonoacrylat und dem hydroxylgruppenhaltigen Methacrylat um Dihydroxyalkyl-Monomethacrylat oder um Dihydroxy-ethoxyalkyl-Monomethacrylat, dessen Glykol-Alkylgruppe 2 bis 6 Kohlenstoffatome enthält, Hydroxy-ethyl-methacrylat, Propandiol-Monomethacrylat, Butandiol-Monomethacrylat, HexandiolMonomethacrylat oder Triethylen-Glycol-Monomethacrylat handelt;
- zwischen 1 und 3 Masse-% einer Mischung aus ethoxyliertem Dioldiacrylat und ethoxyliertem Dioldimethacrylat, bei denen es sich um Polyethylenglykol-Diester handelt, die 2 bis 5 ethoxylierte Funktionen enthalten; und
- zwischen 0,1% und 5 Masse-% Zimtalkohol.

2. Acrylpolymer-Material nach Anspruch 1, **dadurch gekennzeichnet, dass** die Monomermischung mindestens 80%, besser noch mindestens 90% der Masse der Acryl- oder Methacrylmonomere beträgt.

3. Acrylpolymer-Material nach Anspruch 1 oder 2, **dadurch gekennzeichnet, dass** das 2-Phenoxyethylacrylat (2PEA) oder die Mischung aus 2-Phenoxyethylacrylat (2PEA) und 2-Phenoxy-(2-Ethoxy)₄-Acrylat (4PEA) zwischen 70 und 85 Masse-% der Monomermischung bildet.

4. Acrylpolymer-Material nach Anspruch 1, **dadurch gekennzeichnet, dass** die Monomermischung eine Mischung aus 2-Phenoxyethylacrylat (2PEA), oder die Mischung aus 2-Phenoxy-(2-Ethoxy)₄-Acrylat (4PEA) mit 65 bis 75 Masse-% 2PEA und 6 bis 20% 4PEA enthält.

5. Acrylpolymer- Material nach einem der vorangehenden Ansprüche, **dadurch gekennzeichnet, dass** das hydroxylgruppenhaltige Acrylat und das hydroxylgruppenhaltige Methacrylat zusammen zwischen 15 und 30 Masse- % der Monomermischung bilden.

6. Acrylpolymer-Material nach einem der vorangehenden Ansprüche, **dadurch gekennzeichnet, dass** das ethoxylierte Diol-Diacrylat und das ethoxylierte DiolDimethacrylat Tetrathylenglykol-Diacrylat und Tetraethylenglykol-Dimethacrylat sind.

7. Acrylpolymer-Material nach einem der vorangehenden Ansprüche, **dadurch gekennzeichnet, dass** der Zimtalkohol zwischen 0,2% und 2%, und vorzugsweise ungefähr 0,5%, der Masse der Monomermischung bildet.

8. Acryl-Polymermaterial nach einem der vorhergehenden Ansprüche, **dadurch gekennzeichnet, dass** die Monomermischung außerdem ein UV-absorbierendes Monomer enthält.

9. Verfahren zur Herstellung eines Acryl-Polymermaterials nach einem der Ansprüche 1 bis 8, **dadurch gekennzeichnet, dass** es die folgenden Schritte enthält:
eine Monomermischung, die folgende Elemente enthält, wird hergestellt:
- zwischen 50 und 90 Masse-% 2-Phenoxyethylacrylat (2PEA) oder eine Mischung aus 2-Phenoxyethylacrylat (2PEA) und 2-Phenoxy-(2-Ethoxy)₄-Acrylat (4PEA);
- zwischen 8 und 35 Masse-% einer Mischung aus hydroxylgruppenhaltigem Acrylat und hydroxylgruppenhaltigem Methacrylat, wobei es sich bei dem hydroxylgruppenhaltigen Acrylat um ein Dihydroxyalkyl-Monoacrylat oder ein Dihydroxy-Ethoxyalkyl-Monoacrylat handelt, dessen Glykol-Alkylgruppe 2 bis 6 Kohlenstoffatome enthält, wobei es sich bei dem 4-hydroxy-butyl-acrylat, dem Hydroxy-ethyl-acrylat, dem Hexandiol-Monoacrylat oder dem Triethylen-Glycolmonoacrylat und dem hydroxylgruppenhaltigen Methacrylat um Dihydroxyalkyl-Monomethacrylat oder um Dihydroxy-ethoxyalkyl-Monomethacrylat, dessen Glykol-Alkylgruppe 2 bis 6 Kohlenstoffatome enthält, Hydroxy-ethyl-methacrylat, Propandiol-Monomethacrylat, Butandiol-Monomethacrylat, Hexandiolmonomethacrylat oder Triethylen-Glycolmonomethacrylat handelt;
- zwischen 1 und 3 Masse-% einer Mischung aus ethoxyliertem Dioldiacrylat und ethoxyliertem Dioldimethacrylat, bei denen es sich um Polyethylenglykol-Diester handelt, die 2 bis 5 ethoxylierte Funktionen enthalten; und
- zwischen 0,1% und 5 Masse- % Zimtalkohol.
• der genannten Mischung wird mindestens eine Initiatorverbindung zugegeben;
• die genannte Mischung wird radikalreaktiv in einem einzigen Polymerisationsschritt polymerisiert, so dass sich durch diese Polymerisation ein vernetztes, viskoelastisches, flexibles und bei Raumtemperatur verformbares Acryl- oder Methacryl-Copolmer ergibt, das Zimtalkohol enthält.

10. Herstellungsverfahren nach Anspruch 9, **dadurch gekennzeichnet, dass** es außerdem einen Schritt der Reinigung enthält.

11. Weiche Intraokular-Linse zur chirurgischen Implantation in die Linse eines Patienten, als Ersatz für die natürliche Augenlinse, **dadurch gekennzeichnet, dass** sie aus einem Acryl-Polymermaterial gemäß einem der Ansprüche 1 bis 8 hergestellt wird.

## Claims

1. An acrylic polymer material, which is hydrophobic, that is absorbing no more than 5% water at 35°C, suitable for producing flexible intraocular lenses, **characterized in that** it is a copolymer that is crosslinked, viscoelastic, flexible and deformable at room temperature, prepared from a mixture of monomers comprising:
- between 50 and 90 wt % of 2-phenoxy-ethyl acrylate (2PEA), or of a mixture of 2-phenoxy-ethyl acrylate (2PEA) and 2-phenoxy-(2-ethoxy)4-acrylate (4PEA);
- between 8 and 35 wt % of a mixture of hydroxylated acrylate and hydroxylated methacrylate, the hydroxylated acrylate being a dihydroxy-alkyl monoacrylate or a dihydroxy-ethoxy-alkyl monoacrylate whose alkyl chain of the glycol comprises from 2 to 6 carbon atoms, 4-hydroxy-butyl-acrylate, hydroxy-ethyl-acrylate, hexanediol monoacrylate, or triethylene glycol monoacrylate; and the hydroxylated methacrylate being a dihydroxy-alkyl monomethacrylate or a dihydroxy-ethoxy-alkyl monomethacrylate whose alkyl chain of the glycol comprises from 2 to 6 carbon atoms, hydroxy-ethyl-methacrylate, propanediol monomethacrylate, butanediol monomethacrylate, hexanediol monomethacrylate or triethylene glycol monomethacrylate;
- between 1 and 3 wt % of a mixture of ethoxylated diol diacrylate and ethoxylated diol dimethacrylate, that are polyethylene glycol diesters comprising from 2 to 5 ethoxylated functions; and
- between 0.1 wt % and 5 wt % of cinnamic alcohol.

2. The acrylic polymer material according to claim 1, **characterized in that** the mixture of monomers comprises at least 80 wt %, preferably at least 90 wt %, of acrylic or methacrylic monomers.

3. The acrylic polymer material according to claim 1 or 2, **characterized in that** the 2-phenoxy-ethyl acrylate (2PEA) or the mixture of 2-phenoxy-ethyl acrylate (2PEA) and 2-phenoxy-(2-ethoxy)₄-acrylate (4PEA) makes up between 70 and 85 wt % of the mixture of monomers.

4. The acrylic polymer material according to claim 1, **characterized in that** the mixture of monomers comprises a mixture of 2-phenoxy-ethyl acrylate (2PEA) and 2-phenoxy-(2-ethoxy)₄-acrylate (4PEA) with 65 to 75% of 2PEA and 6 to 20% of 4PEA.

5. The acrylic polymer material according to any one of the preceding claims, **characterized in that** the hydroxylated acrylate and the hydroxylated methacrylate together make up between 15 and 30 wt % of the monomer mixture.

6. The acrylic polymer material according to any one of the preceding claims, **characterized in that** the ethoxylated diol diacrylate and the ethoxylated diol dimethacrylate are tetraethylene glycol diacrylate and tetraethylene glycol dimethacrylate.

7. The acrylic polymer material according to any one of the preceding claims, **characterized in that** the cinnamic alcohol makes up between 0.2 wt % and 2 wt %, and more preferably about 0.5 wt %, of the monomer mixture.

8. The acrylic polymer material according to any one of the preceding claims, **characterized in that** the mixture of monomers further comprises a UV-absorbing monomer.

9. A method for manufacturing an acrylic polymer material according to any one of claims 1 to 8, **characterized in that** it comprises the following steps:
• producing a mixture of monomers containing:
- between 50 and 90 wt % of 2-phenoxy-ethyl acrylate (2PEA), or of a mixture of 2-phenoxy-ethyl acrylate (2PEA) and 2-phenoxy-(2-ethoxy)4-acrylate (4PEA);
- between 8 and 35 wt % of a mixture of hydroxylated acrylate and hydroxylated methacrylate, the hydroxylated acrylate being a dihydroxy-alkyl monoacrylate or a dihydroxy-ethoxy-alkyl monoacrylate whose alkyl chain of the glycol comprises from 2 to 6 carbon atoms, 4-hydroxy-butyl-acrylate, hydroxy-ethyl-acrylate, hexanediol monoacrylate, or triethylene glycol monoacrylate; and the hydroxylated methacrylate being a dihydroxy-alkyl monomethacrylate or a dihydroxy-ethoxy-alkyl monomethacrylate whose alkyl chain of the glycol comprises from 2 to 6 carbon atoms, hydroxy-ethyl-methacrylate, propanediol monomethacrylate, butanediol monomethacrylate, hexanediol monomethacrylate or triethylene glycol monomethacrylate;
- between 1 and 3 wt % of a mixture of ethoxylated diol diacrylate and ethoxylated diol dimethacrylate, that are polyethylene glycol diesters comprising from 2 to 5 ethoxylated functions; and
- between 0.1 wt % and 5 wt % of cinnamic alcohol;
• adding at least one initiator compound to said mixture;
• performing radical polymerization of said mixture, in a single polymerization step, so as to obtain, through this polymerization, an acrylic or methacrylic copolymer that is crosslinked, viscoelastic, flexible and deformable at room temperature, and comprising cinnamic alcohol.

10. The manufacturing method according to claim 9, **characterized in that** it further comprises a purification step.

11. A flexible intraocular lens to be surgically implanted in the lens bag of a patient to replace the latter's natural crystalline lens, **characterized in that** it is made from an acrylic polymer material according to any one of claims 1 to 8.
